# EUROPEAN PATENT SPECIFICATION

(11) **EP 3 635 745 B1**
(45) Date of publication and mention of the grant of the patent: **07.10.2026**
(21) Application number: 18731654.2
(22) Date of filing: 24.05.2018
(51) Int. Cl.: G16H 50/20

(54) **ACTIVE UNIPOLAR DRY ELECTRODE OPEN EAR WIRELESS HEADSET AND BRAIN COMPUTER INTERFACE**
AKTIVER, UNIPOLARER, KABELLOSER OFFENER KOPFHÖHRER MIT TROCKENELEKTRODE UND GEHIRN-COMPUTER-SCHNITTSTELLE
CASQUE D'ÉCOUTE OUVERTS SANS FIL À ÉLECTRODE SÈCHE UNIPOLAIRE ACTIVE ET INTERFACE AVEC L'ORDINATEUR CÉRÉBRAL.

(30) Priority: 09.06.2017 US 201715618388
(43) Date of publication of application: 15.04.2020
(73) Proprietor: Drowsy Digital, Inc., Dover DE 19904 (US)
(72) Inventor: FARRELL, Andrew, Framingham Massachusetts 01701-9168 (US); PAETSCH, Christopher R., Framingham Massachusetts 01701-9168 (US); AYERS, Tegan M., Framingham Massachusetts 01701-9168 (US); READ, Jack E., Framingham Massachusetts 01701-9168 (US)
(74) Representative: Cooley (UK) LLP
(86) International application number: PCT/US2018/034401
(87) International publication number: WO 2018/226424

(56) References cited:
- WO-A1-2014/205327
- US-A1- 2015 079 560
- MEREDITH T. CALDWELL ET AL: "Assessment and improvement of sound quality in cochlear implant users", LARYNGOSCOPE INVESTIGATIVE OTOLARYNGOLOGY, vol. 2, no. 3, 1 June 2017 (2017-06-01), pages 119 - 124, XP055729645, ISSN: 2378-8038, DOI: 10.1002/lio2.71
- CHRISTIAN TRONSTAD ET AL: "Electrical measurement of sweat activity", PHYSIOLOGICAL MEASUREMENT., vol. 29, no. 6, 1 June 2008 (2008-06-01), GB, pages S407 - S415, XP055499402, ISSN: 0967-3334, DOI: 10.1088/0967-3334/29/6/S34

## Description

### BACKGROUND

Aspects of the present disclosure relate to wireless technology and, more particularly, to hands-free control of wireless technology.

Wearable devices are electronic devices that are configured to be worn on a user's body and perform various functions, including collecting biometric data for health monitoring. According to an example, electrodes on a wearable device are used to measure electrophysiological signals. These electrophysiological signals can be used to identify patterns in, for example, a user's brain wave activity and identify abnormalities. The abnormalities may be used for diagnostic purposes, as they may be indicative of neurological disorders.

As the types of wearable technology increases and in-view of the projected advancements of wireless communications, including the Internet of Things, it may be beneficial to use collected electrical signals for other purposes.

US 2015/079560 A1 (COWAN JONATHAN DANIEL [US]) 19 March 2015 (2015-03-19) relates to a wearable device measuring brainwaves at two positions and providing neurofeedback based on the difference in the measurements.

CHRISTIAN TRONSTAD ET AL: "Electrical measurement of sweat activity", PHYSIOLOGICAL MEASUREMENT., vol. 29, no. 6, 1 June 2008 (2008-06-01), pages S407-S415, XP055499402, GB ISSN: 0967-3334, DOI: 10.1088/0967-3334/29/6/S34 relates to measurement of sweat activity using skin surface electrodes.

### SUMMARY

All examples and features motioned herein can be combined in any technically possible manner.

According to one example, an open-ear wireless headset including a transducer is used to obtain biologically-relevant information associated with a user. As described in more detail herein, the open-ear wireless headset may take different forms. Regardless of the form taken, the open-ear wireless headset comprises one or more electrodes used to collect biologically-relevant information associated with a user of the open-ear wireless headset.

According to an aspect, a wearable audio product for obtaining biologically-relevant information associated with a user is provided. The audio product comprises a first electrode placed over the user's auditory cortex on a first side of the user's body, a second electrode placed on the first side of the user's body, a first reference electrode placed on the first side of the user's body, a processor coupled to the first, second, and first reference electrode, the processor configured to take one or more actions based on signals received from the first, second, and first reference electrodes, and an electroacoustic transducer coupled to the processor.

According to aspects, the second electrode is placed over one of: the user's occipital lobe, the bridge of the user's nose, or in an ear canal of the user. According to an example, the first reference electrode is placed on the skull of the user behind the user's ear.

According to an example, the audio product performs noise-cancelling between signals received from the first reference electrode and signals received from the first and second electrodes.

According to aspects, the audio product further comprises a third electrode placed over the user's auditory cortex on a second side of the user's body, a fourth electrode placed on the second side of the user's body, and a second reference electrode placed on the second side of the user's body and the processor is further configured to take one or more actions based on signals received from the third, fourth, and second reference electrodes.

According to an example, the audio product applies a first closed feedback loop for signals received from the first side of the body, applies a second closed feedback loop for signals received from the second side of the body, and removes noise from the received signals based on the first and second closed feedback loops.

According to aspect, the audio product determines a state of the user based on the signals received via at least one of the first electrode, the second electrode, or the first reference electrodes and the audio product takes one or more actions based on the determined state.

According to an example, the audio product determines a drop in impedance of signals received via at least one of the first electrode, the second electrode, or the first reference electrode and in response to the determined drop in impedance, the audio product determines the user is stressed. As described in more detail herein, the product determines with an increased confidence that the user is stressed. For example, the drop in impedance increases the confidence or likelihood that the user is stressed.

According to an aspect, the audio product tracks a type of music played in response to determining the user is stressed. In response to determining the user is stressed, the audio product recommends at least one of a song or melody based on the tracked type of music.

According to an example, the audio product of determines a decreased rate of blinking of the user based on signals received via at least one of the first and second electrodes. In response to the determined decreased rate of blinking, the product determines the user is focused. As described in more detail herein, the product determines with an increased confidence that the user is trying to focus. For example, the determined decreased rate of blinking increases the confidence or likelihood that the user is trying to focus.

According to an aspect, the audio product determines (with an increased confidence) the user is focused and, in response, recommends a certain song or melody.

According to an example, the audio product determines the state of the user by measuring jaw movements based on signals received from at least one of the first electrode, the second electrode, or the first reference electrode. In response to the measured jaw movements, the audio product determines the user is talking. According to an example, signals received from the auditory cortex in combination determined jaw movements are used to determine, with increased confidence or increased likelihood, the user is talking.

According to an aspect, the audio product adjusts a volume of music played in response to determining the user is talking.

According to an example, the audio product triggers automatic speech recognition (ASR) in response to determining the user is talking. According to an example, the audio product enters a low power mode in response to determining the user is not talking.

According to an aspect, a method for obtaining biologically-relevant information associated with a user from wearable audio product including an electroacoustic transducer is provided. The method generally includes receiving electrical signals via a first electrode placed over the user's auditory cortex on a first side of the user's body, receiving electrical signals via a second electrode placed on the first side of the user's body, receiving electrical signals via a first reference electrode placed on the first side of the user's body, and taking one or more actions based, at least in part, on the signals received from the first, second, and reference electrodes.

According to an example, the second electrode is placed over one of the user's occipital lobe, the bridge of the user's nose, or in an ear canal of the user.

According to an aspect, a wearable audio product including an electroacoustic transducer for obtaining biologically-relevant information associated with a user is provided. The wearable audio product comprises a first electrode, a second electrode, and a first reference electrode each placed on a first side of the user's body. The audio product comprises a third electrode, a fourth electrode, and a second reference electrode each placed on a second side of the user's body. The audio product comprises a processor coupled to the first, second, third, fourth, first reference, and second reference electrodes, wherein the processor is configured perform noise canceling on signals received via the first, second, third, and fourth electrodes and control the device based on the noise-canceled signals.

According to an example, the first and third electrodes are placed over the first and second side of the auditory cortex of the user, respectively. According to an example, the second and fourth electrodes are placed over one of: the first and second side of the user's occipital lobe, the first and second side of a bridge of the user's nose, or in each ear canal of the user.

### BRIEF DESCRIPTION OF THE DRAWINGS

FIG. 1 is a block diagram of a wireless device.
FIGs. 1A-1C illustrate example form factors for the wireless device.
FIG. 2 is a flow diagram illustrating example steps performed by a wearable audio product for obtaining biologically-relevant information associated with a user of the product.
FIG. 3 illustrates an example of processing signals received from the first to fourth data-collecting electrodes.

### DETAILED DESCRIPTION

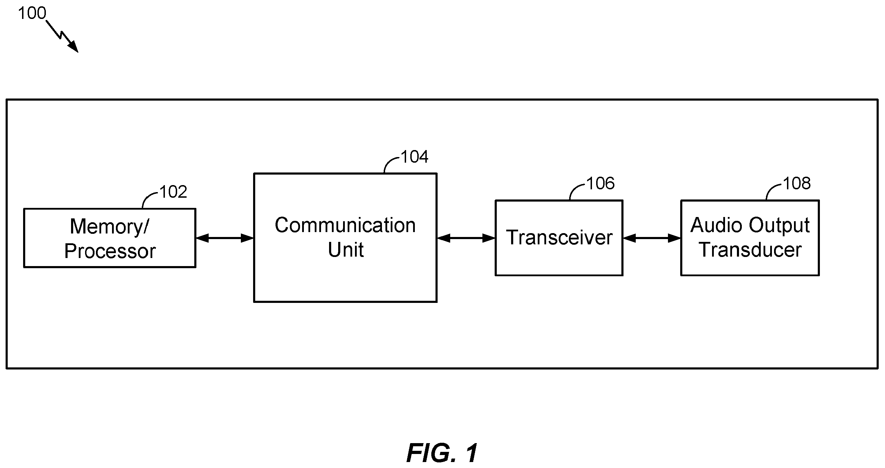

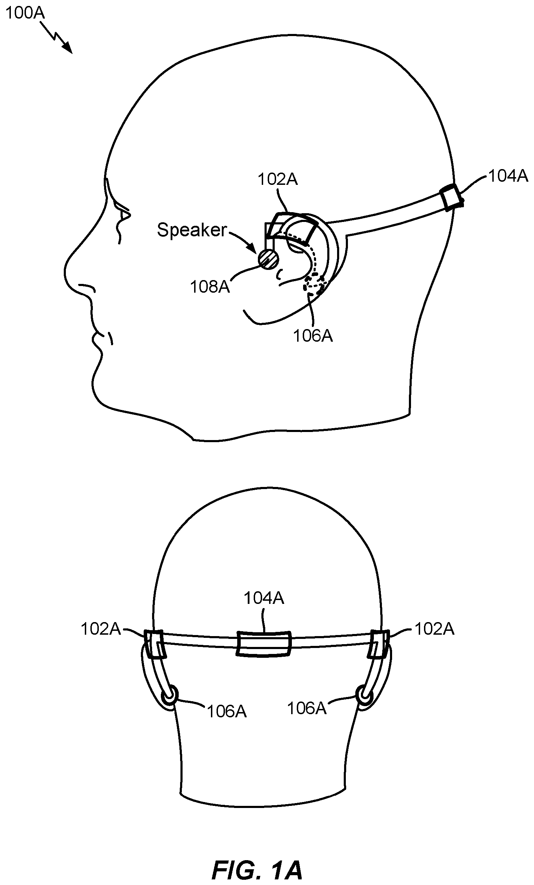

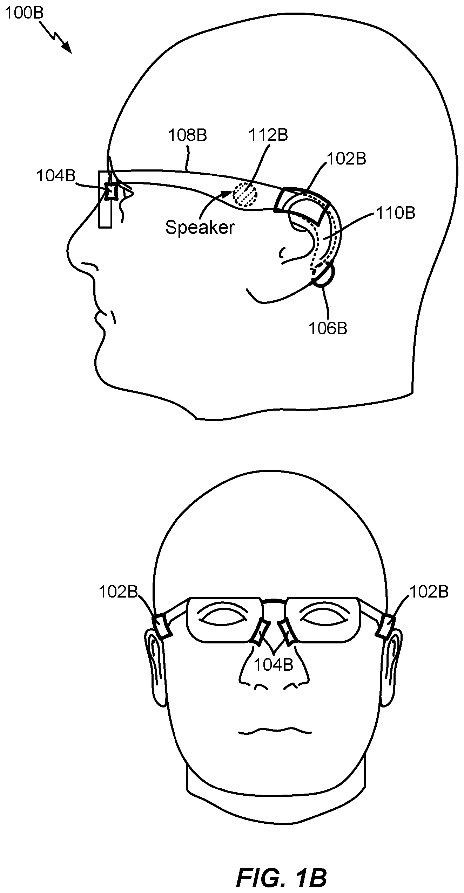

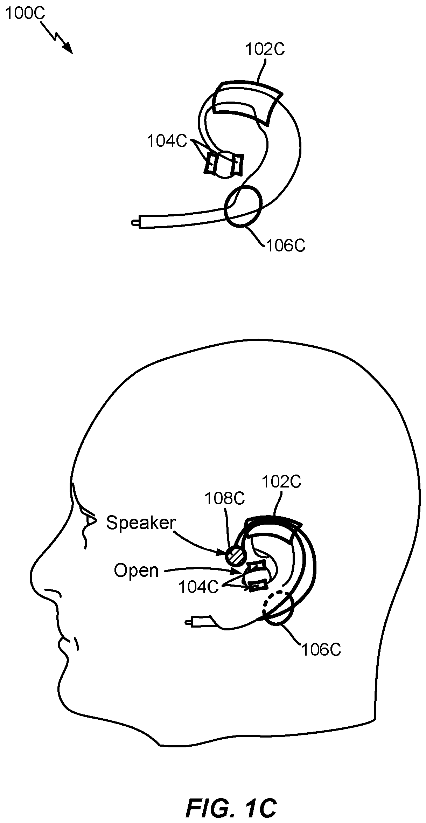

Aspects of the present disclosure relate to a single integrated wearable product configured to collect biologically-relevant information associated with a user of the wearable product. The wearable product may take one or more actions to intelligently control itself based on the collected information. The collected information is used to determine a state of the user. As described below, a state of a user is not absolute. Accordingly, the collected information is used to determine, for example, with an increased confidence level or an increased likelihood, a particular state of a user. Example states of a user include stressed, not stressed, focused, not focused, talking, eating, or minimal jaw movement. The determined state of the user is used to trigger actions of the wearable device, such as changing a type of music played, changing the volume of the music, acknowledging a notification, or triggering automatic speech recognition (ASR). According to one example, the wearable product is an audio product. The audio product may take one of several form factors. Example form factors are provided in FIGs. 1A, 1B, and 1C.

FIG. 1 illustrates example components of a wireless device 100, for example an integrated wearable product such as an audio product. According to one example, the audio product is an open-ear wireless headset including a transducer 108. The wireless device includes a memory and processor 102, communication unit 104, transceiver 106, and audio output transducer 108. The memory may include Read Only Memory (ROM), a Random Access Memory (RAM), and/or a flash ROM. The memory stores program code for controlling the memory and processor 102. The memory and processor 102 control the operations of the wireless device 100. Any or all of the components in FIG. 1 may be combined into multi-function components.

The processor 102 controls the general operation of the wireless device 100. For example, the processor 102 performs process and control for audio and/or data communication. In addition to the general operation, the processor 102 initiates processing signals received from electrodes of the wireless device 100 to control the wireless device 100 as described in more detail herein.

The communication unit 104 facilitates a wireless connection with one or more other wireless devices. For example, the communication unit 104 may include one or more wireless protocol engines such as a Bluetooth engine. While Bluetooth is used as an example protocol, other communication protocols may also be used. Some examples include Bluetooth Low Energy (BLE), Near Field Communications (NFC), IEEE 802.11, or other local area network (LAN) or personal area network (PAN) protocols.

The transceiver 106 transmits and receives information via one or more antennae to exchange information with one or more other wireless devices. According to aspects, the transceiver 106 includes one or more microphones. The transceiver 106 is not necessarily a distinct component. The transceiver 106 may be implemented entirely in software executed by the communication unit 104.

The audio output transducer 108 may be also known as a driver or speaker. In some examples, more than one output transducer is used. The transducer converts electrical signals into sound and converts sound into electrical signals.

The wireless device 100 includes multiple electrodes, configured to receive electrical signals from a user of the wireless device. The electrodes may be active, unipolar, dry electrodes. According to one example, the wireless device has two data-collecting electrodes and one reference electrode. The first and second data-collecting electrodes and the first reference electrode each contact a first side of the user's body. For example, each of these three electrodes contacts either a left side or right side of the user's body. Any of the data-collecting electrodes and reference electrodes can include a cluster of electrodes positioned in the specific areas described herein. The cluster of electrodes in the specific location may be viewed as an electrode.

The first electrode is placed over the user's auditory cortex on the same side of the user's body as the second electrode and the first reference electrode. The second electrode is placed over one of the user's occipital lobe (for example, as illustrated in FIG. 1A), the bridge of the user's nose (for example, as illustrated in FIG. 1B), or contacts an ear canal of the user (for example, as illustrated in FIG. 1C). According to an example, the first reference electrode is placed on the skull of the user, behind the user's ear.

FIG. 1A illustrates an example form factor 100A wherein the open-ear wireless headset includes a band covering part of the user's occipital lobe. The band stretches from above and behind the user's right ear, behind the back of the head covering the occipital lobe, to above and behind the user's left hear. The form factor 100A includes at least one speaker 108A. According to an example, a speaker 108A hangs over or is proximate to at least a portion of the user's ear.

According to an example, the first electrode 102A is placed on the band, over the user's ear on one side of the user's body to monitor the auditory cortex. The second electrode 104A is placed on the band, behind the back of the head on the same side of the user's body as the first electrode. As illustrated, the second electrode 104A is placed over the user's occipital lobe. The first reference electrode 106A is placed on any other portion of the form factor 100A. The pressure exerted by the band on the user's head enables the first and second data-collecting electrodes 102A, 104A and the first reference electrode 106A to contact the user's head and measure electrical signals from the occipital lobe, auditory cortex, and reference location.

FIG. 1B illustrates an example form factor 100B wherein the second electrode is placed on the bridge of the user's nose. According to the illustrated example, form factor 100B comprises glasses. Each temple 108B and temple tip 110B of the glasses 100B rest over an ear of the user. According to an example, a speaker 112B is placed inside a temple 108B. For example, the temple 108B has an opening towards the user's body. The speaker 112B is located in this opening.

The first electrode 102B configured to monitor the auditory cortex is placed on the temple 108B or temple tip 110B. The second electrode 104B is placed on the bridge or nose pad of the glasses. The first reference electrode 106B is placed on any other portion of the glasses 110B or on a band (such as one similar to 100A) , which connects each of the temple tips 110B and covers the user's occipital lobe.

FIG. 1C illustrates an example form factor 100C wherein the second electrode is placed in an ear canal of the user. According to the illustrated example, form factor 100C comprises an headset. The headset is a C-shaped headset, wherein a "C" shaped clip hooks behind a user's ear. The headset may be an open ear headset. The ear tip is inserted inside the user's ear. According to an aspect, the ear tip is made of a flexible material, which conforms to the shape of the user's ear and creates a gentle seal with the ear canal. Similar to form factor 100A illustrated in FIG. 1A, speaker 108C hangs over or is proximate to at least a portion of the user's ear.

The first data-collecting electrode 102C is place on the "C" shaped portion behind the user's ear to monitor signals from the auditory cortex. According to aspects, the first data-collecting electrode 102C monitors signals such as an electrooculogram (EOG), electrocardiogram (EKG), electromyogram (EMG), or electroencephalogram (EEG). The second data-collecting electrode 104C is placed on the ear tip. The second data-collecting electrode 104C monitors signals such as EOG, EKG, EMG, or EEG. The first reference electrode 106C is placed on any other portion of the headset 100C or on a band (such as one similar to 100A), which connects each of the left and right ear pieces of the headset and covers the occipital lobe. According to an example, the first reference electrode 106C is wirelessly connected to the first and second data-collecting electrodes 102C and 104C. Accordingly, the band does not necessarily physically connect the left and right ear pieces of the headset.

According to an example, the wireless device implemented using any form factor also has third and fourth data-collecting electrodes and a second reference electrode. The third and fourth data-collecting electrodes and the second reference electrode each contact a same side of the user's body. Assuming the first and second data-collecting electrodes and the first reference electrode each contact a left side of the user's body, the third and fourth data-collecting electrodes and the second reference electrode each contact a right side of the user's body.

The third electrode is placed over the user's auditory cortex on the same side of the user's body as the fourth electrode and the second reference electrode. The fourth electrode is placed over one of the user's occipital lobe, the bridge of the user's nose, or in an ear canal of the user.

As described above, when the fourth electrode is placed over the user's occipital lobe, the wireless device may comprise a band that stretches from above and behind the user's ear on a first side of the body, behind the back of the head covering the occipital lobe of the user, to above and behind the user's ear on a second side of the body. Assuming the first and second data-collecting electrodes and the first reference electrode each contact a left side of the user's body, the third electrode may be placed above the user's right ear to monitor the auditory cortex. The fourth electrode may be placed behind the back of the head on the right side of the user's body to monitor the occipital lobe. The second reference electrode is placed on any other portion of the band. As described above, the pressure exerted by the band on the user's head enables the electrodes to contact the user's head and measure electrical signals from the auditory cortex, occipital lobe, and reference location.

Similarly, when the fourth electrode is placed on the bridge of the user's nose, the wireless device may comprise glasses. Assuming the first and second data-collecting electrodes and the first reference electrode each contact a left side of the user's body, the third and fourth data-collecting electrodes and second reference electrode are placed on the right side of the user's body. Accordingly, the third electrode is placed on the right temple or right temple tip of the glasses. The fourth electrode is placed on the right side of the bridge or on the right nose pad of the glasses. The second reference electrode is placed on any other portion of the glasses contacting the right side of the user's body or on a right side of the band, which connects each of the temple tips and covers the occipital lobe of the user.

When the fourth electrode is place in an ear canal of the user, the wireless device may comprise a headset. According to an example, the headset is a C-shaped headset, such as an open-ear headset. Assuming the first and second data-collecting electrodes and the first reference electrode each contact a left side of the user's body, the third and fourth data-collecting electrodes and the second reference electrode are placed on a portion of the headset contacting the rights side of the user's body. According to an example, the third data-collecting electrode is placed on the "C" shaped portion behind the user right ear, the fourth data-collecting electrode is placed on the right ear, and the second reference electrode is placed on any other portion of the headset or on a band contacting the right side of the user, wherein the band connects each of the left and right ear pieces of the headset and covers the user's occipital lobe.

Each of the first to fourth data-collecting electrodes and first and second reference electrodes includes one or multiple electrodes. According to an example two, three, or any integer number of electrodes may be clustered in the areas specified above (102A-106A, 102B-106B, and 102C-106C). The cluster of electrodes in one location may be collectively referred to as an electrode.

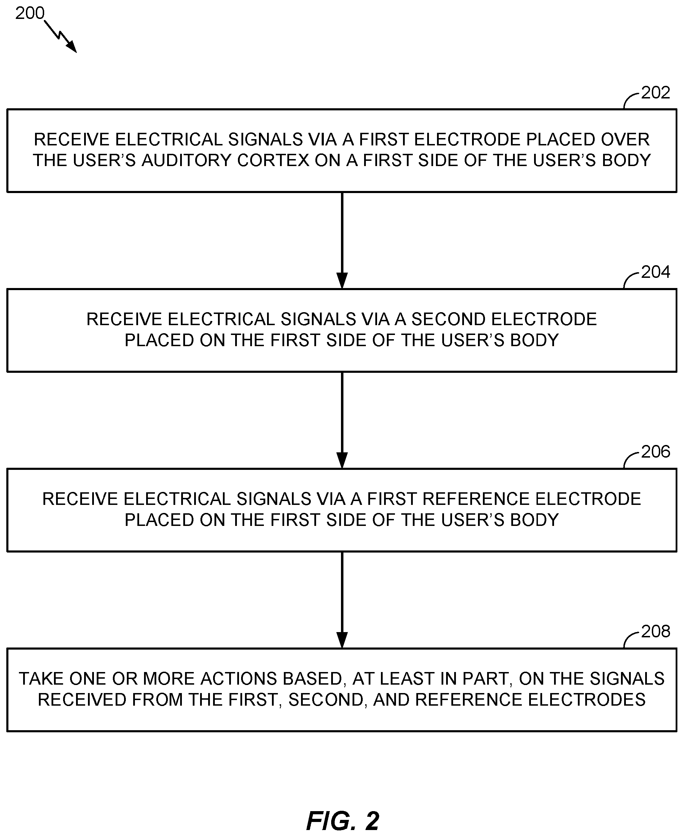

FIG. 2 illustrates example operations 200 performed by a wearable audio product. The wearable audio product has one or more of the components illustrated in FIG. 1.

At 202, the audio product receives electrical signals via a first electrode placed over the user's auditory cortex on a first side of the user's body. At 204, the audio product receives electrical signals via a second electrode placed on the first side of the user's body. The second electrode is placed over one of the user's occipital lobe, the bridge of the user's nose, or in an ear canal of the user.

At 206, the audio product receives electrical signals via a first reference electrode placed on the first side of the user's body. According to an example, the reference electrode is placed at any location on the audio product that is different than the location of the first and second electrodes.

At 208, the audio product takes one or more actions based, at least in part, on the signals received from the first, second, and reference electrodes. According to an example, the audio product also receives electrical signals via a third electrode placed over the user's auditory cortex on a second side of the user's body, a fourth electrode placed on the second side of the user's body, and a second reference electrode placed on the second side of the user's body, wherein the all of the described electrodes are part of the audio product. In an effort to remove noise from the collected signals, the audio product performs noise-cancelling on signals received from the four data-collecting electrodes.

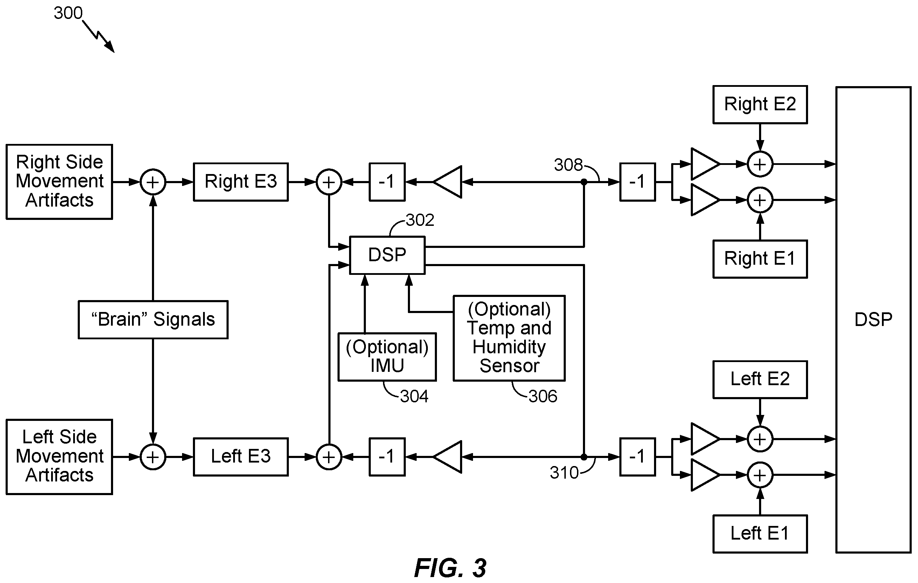

FIG. 3 illustrates an example 300 of signal processing performed by the wearable audio product. Left E1 and Left E2 refer to the first and second data-collecting electrodes and Right E1 and Right E2 refer to the third and fourth data-collecting electrodes. Left E3 refers to the first reference electrode and Right E3 refers to the right reference electrode.

The digital signal processor (DSP) 302 processes a combined signal from the left side movement artifacts, the brain, and Left E3. DSP 302 also processes a combined signal from the right side movement artifacts, brain signals, and Right E3. The DSP 302 performs short-time Fourier transform and compares the combined signal from the left and right side and outputs a difference per side at 308 and 310. In an effort to remove noise, the DSP 302 optionally receives an output from an inertial measurement unit (IMU) 304 and/or from a temperature and humidity sensor 306. The IMU 304 accounts for entire body movements such as running or walking. The temperature and humidity sensor 306 may be used by the DSP to adjust a gain based on the ambient temperate and humidity.

The wearable audio product applies one closed feedback loop for signals collected from each side of the user's body in an effort to remove noise from the collected signals. The feedback loop associated with the left side of the user's body is between Left E3 against Left E1 and Left E2. The feedback loop associated with the right side of the user's body is between Right E3 against Right E1 and Right E2.

Signals received from each side of the body are cross-correlated and noise may be further reduced by comparing the difference signal against an audio stream, in an effort to remove noise that is induced or coupled from the speakers or wires in the audio product.

Using signals received from at least one side of the body (for example, Left E1, Left E2, and Left E3) and/or using noise-reduced signals from at least one side of the body, the audio product determines, with increased confidence, a state of the user. Example states of the user include determining the user is stressed (or not stressed), focused (or not focused), or moving a jaw (or not moving/having minimal jaw movement). As states of a user are not absolute, the signals received via the electrodes are used to determine an increased likelihood the user is in a particular state. Furthermore, according to an example, other biolsignals such as the user's heart rate, respiration rate, movement from IMU, etc. are used in conjunction with the signals collected using the above-described electrodes to determine an increased likelihood the user is in a particular state.

The audio product determines a user is stressed (or not stressed) by measuring overload in the user's auditory cortex (from the first or third data-collecting electrode). The user's autonomous nervous system may cause sweat glands to secrete sweat. The conductive sweat may cause a drop in impedance measured by data- collected electrodes, the reference electrodes, or a combination thereof.

The audio product determines a user is focused (or not focused) by measuring occipital cortex overload in, for example, the alpha band (8-13 Hz). Occipital cortex overload may be measured using the electrode placed over the occipital band (for example, the second or fourth data collecting electrode). An overload of measured activity in the occipital cortex indicates the user is focused.

The audio product determines a user is focused (or not focused) by measuring a rate of blinking. Blinking rates are measured from EMG or EOG signals. EMG or EOG signals may be measured using at least one of the first to fourth data-collecting electrodes. The processor may compare signals from both the left and right sides of the user to differentiate between a blink versus a wink or eye twitch. The audio product may collect data over time to determine the user's average blink rate. According to aspects, the audio product uses a moving average filter for signals obtained via the occipital cortex for determining eye movement. A decreased rate of blinking or eye movement indicates the user is focused.

The audio product determines a user is moving a jaw by measuring EMG signals using any of the above-described electrodes. According to an example, jaw movements indicate the use is talking or chewing. According to an example, signals received from the auditory cortex in combination determined jaw movements are used to determine, with increased confidence, the user is talking.

The state of the user determined by the audio product is used to intelligently control the audio product itself. According to one example, the audio product enters a low-power state when the user is not talking. The ASR engine listens using microphones of the product when the user is talking. According to an example, the ASR engine listens for the Wake Up Work (WUW), which triggers ASR. This increases a user's privacy because the audio product is not always listening to the user and reduces battery consumption due to decreased high frequency sampling. Over time, a neural network may be trained to learn the user's EMG pattern correlated with a WUW thereby reducing false voice personal assistant (VPA) triggers.

According to an example, the audio product dynamically adjusts the volume of music played when it determines the user is talking. Accordingly, the user does not have to manually decrease or mute the music. When the audio product determines the user is no longer speaking, it increases the volume. In this manner, the audio product enables seamless, intelligent, hands-free operations of the audio product.

According to an example, the audio product correlates certain eye movements with actions such as changing the volume, changing songs, or acknowledging notifications. Accordingly, EMG readings from blinking, which are discemable based on signals received from signals received via the auditory electrodes, enable hands-free operation of the audio product.

According to another example, the electrodes may discern when a tip of the tongue, having an overall negative charge, touches the inside of the user's cheek. The audio product may correlate certain tongue-to-cheek patterns with actions such as changing the volume, changing songs, or acknowledging notifications.

After determining the user is stressed (or not stressed), the audio product may track what songs the user listens to while stressed (or not stressed). The audio product may also track the songs the user listens to while focused (or not focused). According to an example, the audio product learns what songs or melodies the user listens to when transitioning from a stressed state to a not stressed state. According to an aspect, the audio product transmits a determined state of the user to an external cloud. Based on the songs a user listens to while focused (or not focused), the cloud leams which songs or melodies the user listens to when transitioning form a stressed state to a not stressed state. The cloud communicates song and/or melody recommendations to the audio product. Accordingly, the audio product may accurately recommend or play songs base on the user's determined state.

The audio product may enhance personalization by training neural networks to leam the phrasing and melodies associated with the user's frequently-played songs. In response to determining the user is stressed or focused, the audio product may play one or more songs having similar phrasing or melodies.

As described herein, multiple electrodes on an audio product are used to collect biologically-relevant information associated with a user. The collected information is used to ascertain a state of the user. Based on the determined state of the user, the audio product takes one or more actions to intelligently adapt or respond to the user. In this manner, the audio product provides personalized, hands-free control.

## Claims

1. A wearable audio product (100) for obtaining biologically-relevant information associated with a user and for controlling itself based on the obtained biologically-relevant information, comprising:
a first electrode (102A,102B,102C) placed over the user's auditory cortex on a first side of the user's body;
a second electrode (104A,104B,104C) placed on the first side of the user's body;
a first reference electrode (106A,106B,106C) placed on the first side of the user's body;
a third electrode placed over the user's auditory cortex on a second side of the user's body, the third electrode configured to monitor signals;
a fourth electrode placed on the second side of the user's body;
a second reference electrode placed on the second side of the user's body;
a processor (102) coupled to the first, second, third, fourth, first reference, and second reference electrodes, the processor configured to:
process a combined signal from first side movement artifacts, the user's brain, and the first reference electrode;
process a combined signal from second side movement artifacts, the user's brain, and signals from the second reference electrode;
compare the combined signal from the first side and the combined signal from the second side and output a difference per side;
reduce noise in signals received from the first, second, third, and fourth electrodes using at least the difference per side;
determine a state of the user using the noise-reduced signals from the first, second, third, and fourth electrodes; and
take one or more actions to control operation of the wearable audio product based on the state of the user; and
an electroacoustic transducer (108) coupled to the processor.

2. The audio product (100) of claim 1, wherein taking the one or more actions to control operation of the wearable audio product comprises any one of: changing a type of music played, changing a volume of music played, acknowledging a notification, triggering automatic speech recognition.

3. The audio product (100) of claim 1, wherein the second electrode is placed over one of: the user's occipital lobe, the bridge of the user's nose, or in an ear canal of the user.

4. The audio product (100) of claim 1, wherein the first reference electrode is placed on the skull of the user behind the user's ear.

5. The audio product (100) of claim 1, wherein the processor is configured to reduce the noise in signals received from the first, second, third, and fourth electrodes by:
applying a first closed feedback loop for signals received from the first side of the body, the first closed feedback being between the first reference electrode against the first electrode and the second electrode;
applying a second closed feedback loop for signals received from the second side of the body, the second closed feedback being between the second reference electrode against the third electrode and the fourth electrode; and
removing noise from the received signals based on the first and second closed feedback loops, wherein removing the noise comprises cross-correlating the signals received from the first side of the body with the signals received from the second side of the body.

6. The audio product (100) of claim 1, wherein determining the state of the user comprises:
determining a drop in impedance of signals received from at least one of the first electrode, the second electrode, the third electrode, or the fourth electrode; and
in response to the determined drop in impedance, determining the user is stressed.

7. The audio product (100) of claim 6, wherein taking one or more actions based on the determined state comprises:
tracking a type of music played in response to determining the user is stressed; and
in response to determining the user is stressed, recommending at least one of a song or melody based on the tracked type of music.

8. The audio product (100) of claim 1, wherein at least one of the first, second, third, or fourth electrodes is configured to measure electromyography or electrooculography signals, and determining the state of the user comprises:
determining a decreased rate of blinking of the user based on the electromyography or electrooculography signals received from the at least one of the first, second, third, or fourth electrodes; and
in response to the determined decreased rate of blinking, determining the user is focused.

9. The audio product (100) of claim 8, wherein taking one or more actions based on the determined state comprises:
in response to determining the user is focused, recommending a certain song or melody.

10. The audio product (100) of claim 1, wherein at least one of the first, second, third, or fourth electrodes is configured to measure electromyography signals, and determining the state of the user comprises:
determining jaw movements based on the electromyography signals received from the at least one of the first, second, third, or fourth electrodes; and
using signals received from the auditory cortex and the determined jaw movements, determining the user is talking.

11. The audio product (100) of claim 10, wherein taking one or more actions based on the determined state comprises:
adjusting a volume of music played by the audio product in response to determining the user is talking.

12. The audio product (100) of claim 10, further comprising a microphone, wherein taking one or more actions based on the determined state comprises:
triggering automatic speech recognition (ASR) by listening using the microphone in response to determining the user is talking

13. The audio product (100) of claim 12, wherein taking one or more actions based on the determined state further comprises:
entering a low power mode in response to determining the user is not talking.

14. The audio product (100) of claim 1, wherein the first, second, third and fourth electrodes and the first and second reference electrodes each measure at least two of electrooculogram (EOG), electrocardiogram (EKG), electromyogram (EMG), or electroencephalogram (EEG) data, and determining the state of the user comprises:
using the at least two of EOG, EKG, EMG, or EEG data to monitor an overload in the user's auditory cortex, an overload in the user's occipital cortex, a rate of blinking of the user, or jaw movements of the user.

15. A method for obtaining biologically-relevant information associated with a user from wearable audio product (100) including an electroacoustic transducer (108) and for the wearable audio product controlling itself based on the obtained biologically-relevant information, comprising:
receiving (202) electrical signals from a first electrode (102A,102B,102C) placed over the user's auditory cortex on a first side of the user's body;
receiving (204) electrical signals from a second electrode (104A,104B,104C) placed on the first side of the user's body;
receiving (206) electrical signals from a first reference electrode (106A,106B,106C) placed on the first side of the user's body;
receiving electrical signals from a third electrode placed over the user's auditory cortex on a second side of the user's body;
receiving electrical signals from a fourth electrode placed on the second side of the user's body;
receiving electrical signals from a second reference electrode placed on the second side of the user's body;
processing a combined signal from first side movement artifacts, the user's brain, and the first reference electrode;
processing a combined signal from second side movement artifacts, the user's brain, and signals from the second reference electrode;
comparing the combined signal from the first side and the combined signal from the second side and output a difference per side;
reducing noise in the electrical signals received from the first, second, third, and fourth electrodes using at least the difference per side;
determining a state of the user using the noise-reduced signals from the first, second, third, and fourth electrodes; and
taking (208) one or more actions to control operation of the wearable audio product based on the state of the user.

## Patentansprüche

1. Am Körper tragbares Audioprodukt (100) zum Erhalten von biologisch relevanten Informationen, die mit einem Benutzer verknüpft sind, und zur Selbststeuerung basierend auf den erhaltenen biologisch relevanten Informationen, umfassend:
eine erste Elektrode (102A, 102B, 102C), die über dem auditiven Cortex des Benutzers auf einer ersten Seite des Benutzerkörpers platziert ist;
eine zweite Elektrode (104A, 104B, 104C), die auf der ersten Seite des Benutzerkörpers platziert ist;
eine erste Referenzelektrode (106A, 106B, 106C), die auf der ersten Seite des Benutzerkörpers platziert ist;
eine dritte Elektrode, die über dem auditiven Cortex des Benutzers auf einer zweiten Seite des Benutzerkörpers platziert ist, wobei die dritte Elektrode dazu ausgelegt ist, Signale zu überwachen;
eine vierte Elektrode, die auf der zweiten Seite des Benutzerkörpers platziert ist;
eine zweite Referenzelektrode, die auf der zweiten Seite des Benutzerkörpers platziert ist;
einen Prozessor (102), der an die erste, zweite, dritte, vierte Elektrode, die erste Referenzelektrode und die zweite Referenzelektrode gekoppelt ist, wobei der Prozessor hierzu ausgelegt ist:
Verarbeiten eines kombinierten Signals von erstseitigen Bewegungsartefakten, dem Gehirn des Benutzers und der ersten Referenzelektrode;
Verarbeiten eines kombinierten Signals von zweitseitigen Bewegungsartefakten, dem Gehirn des Benutzers und Signalen von der zweiten Referenzelektrode;
Vergleichen des kombinierten Signals von der ersten Seite und dem kombinierten Signal von der zweiten Seite und Ausgeben einer Differenz pro Seite;
Reduzieren von Rauschen in Signalen, die von der ersten, zweiten, dritten und vierten Elektrode empfangen werden, wenigstens unter Verwendung der Differenz pro Seite;
Bestimmen eines Zustands des Benutzers unter Verwendung der rauschreduzierten Signale von der ersten, zweiten, dritten und vierten Elektrode; und
Durchführen einer oder mehrerer Aktionen zum Steuern des Betriebs des am Körper tragbaren Audioprodukts basierend auf dem Zustand des Benutzers; und
einen elektroakustischen Wandler (108), der an den Prozessor gekoppelt ist.

2. Audioprodukt (100) nach Anspruch 1, wobei das Durchführen der ein oder mehreren Aktionen zum Steuern des Betriebs des am Körper tragbaren Audioprodukts eines hiervon umfasst: Ändern einer Art von Musik, die wiedergegeben wird, Ändern einer Lautstärke wiedergegebener Musik, Bestätigen einer Benachrichtigung, Auslösen einer automatischen Spracherkennung.

3. Audioprodukt (100) nach Anspruch 1, wobei die zweite Elektrode über einem hiervon platziert ist: dem Okzipitallappen des Benutzers, dem Nasenrücken des Benutzers, oder in einem Gehörgang des Benutzers.

4. Audioprodukt (100) nach Anspruch 1, wobei die erste Referenzelektrode am Schädel des Benutzers hinter dem Ohr des Benutzers platziert ist.

5. Audioprodukt (100) nach Anspruch 1, wobei der Prozessor dazu ausgelegt ist, das Rauschen in Signalen, die von der ersten, zweiten, dritten und vierten Elektrode empfangen werden, hierdurch zu reduzieren:
Anwenden einer ersten geschlossenen Rückkopplungsschleife für Signale, die von der ersten Seite des Körpers empfangen werden, wobei die erste geschlossene Rückkopplung zwischen der ersten Referenzelektrode gegen die ersten Elektrode und die zweite Elektrode erfolgt;
Anwenden einer zweiten geschlossenen Rückkopplungsschleife für Signale, die von der zweiten Seite des Körpers empfangen werden, wobei die zweite geschlossene Rückkopplung zwischen der zweiten Referenzelektrode gegen die dritte Elektrode und die vierte Elektrode erfolgt; und
Entfernen von Rauschen aus den empfangenen Signalen basierend auf der ersten und zweiten geschlossenen Rückkopplungsschleife, wobei das Entfernen des Rauschens das Kreuzkorrelieren der Signale, die von der ersten Seite des Körpers empfangen werden, mit den Signalen, die von der zweiten Seite des Körpers empfangen werden, umfasst.

6. Audioprodukt (100) nach Anspruch 1, wobei das Bestimmen des Zustands des Benutzers dies umfasst:
Bestimmen eines Abfalls der Impedanz von Signalen, die von wenigstens einem von der ersten Elektrode, der zweiten Elektrode, der dritten Elektrode oder der vierten Elektrode empfangen werden; und
in Reaktion auf den bestimmten Abfall der Impedanz, Bestimmen, dass der Benutzer unter Stress steht.

7. Audioprodukt (100) nach Anspruch 6, wobei das Durchführen einer oder mehrerer Aktionen basierend auf dem bestimmten Zustand dies umfasst:
Verfolgen einer Art von Musik, die in Reaktion auf das Bestimmen wiedergegeben wird, dass der Benutzer unter Stress steht; und
in Reaktion auf das Bestimmen, dass der Benutzer unter Stress steht, Empfehlen wenigstens eines von einem Lied oder einer Melodie basierend auf der verfolgten Art von Musik.

8. Audioprodukt (100) nach Anspruch 1, wobei wenigstens eine von der ersten, zweiten, dritten oder vierten Elektrode dazu ausgelegt ist, Elektromyographie- oder Elektrookulographiesignale zu messen, und das Bestimmen des Zustands des Benutzers dies umfasst:
Bestimmen einer verringerten Blinzelrate des Benutzers basierend auf den Elektromyographie- oder Elektrookulographiesignalen, die von wenigstens einer von der ersten, zweiten, dritten oder vierten Elektrode empfangen werden; und
in Reaktion auf die bestimmte verringerte Blinzelrate, Bestimmen, dass sich der Benutzer konzentriert.

9. Audioprodukt (100) nach Anspruch 8, wobei das Durchführen einer oder mehrerer Aktionen basierend auf dem bestimmten Zustand dies umfasst:
in Reaktion auf das Bestimmen, dass sich der Benutzer konzentriert, Empfehlen eines bestimmten Liedes oder einer bestimmten Melodie.

10. Audioprodukt (100) nach Anspruch 1, wobei wenigstens eine von der ersten, zweiten, dritten oder vierten Elektrode dazu ausgelegt ist, Elektromyographiesignale zu messen, und das Bestimmen des Zustands des Benutzers dies umfasst:
Bestimmen von Kieferbewegungen basierend auf den Elektromyographiesignalen, die von wenigstens einer von der ersten, zweiten, dritten oder vierten Elektrode empfangen werden; und
unter Verwendung von Signalen, die von dem auditiven Cortex empfangen werden, und den bestimmen Kieferbewegungen, Bestimmen, dass der Benutzer gerade spricht.

11. Audioprodukt (100) nach Anspruch 10, wobei das Durchführen einer oder mehrerer Aktionen basierend auf dem bestimmten Zustand dies umfasst:
Anpassen einer Lautstärke von Musik, die durch das Audioprodukt wiedergegeben wird, in Reaktion auf das Bestimmen, dass der Benutzer gerade spricht.

12. Audioprodukt (100) nach Anspruch 10, ferner umfassend ein Mikrofon, wobei das Durchführen einer oder mehrerer Aktionen basierend auf dem bestimmten Zustand dies umfasst:
Auslösen einer automatischen Spracherkennung (Automatic Speech Recognition, ASR) durch Abhören unter Verwendung des Mikrofons in Reaktion auf das Bestimmen, dass der Benutzer gerade spricht.

13. Audioprodukt (100) nach Anspruch 12, wobei das Durchführen einer oder mehrerer Aktionen basierend auf dem bestimmten Zustand ferner dies umfasst:
Eintreten in einen Niedrigenergiemodus in Reaktion auf das Bestimmen, dass der Benutzer gerade nicht spricht.

14. Audioprodukt (100) nach Anspruch 1, wobei die erste, zweite, dritte und vierte Elektrode und die erste und zweite Referenzelektrode jeweils wenigstens zwei von Elektrookulogramm- (EOG), Elektrokardiogramm- (EKG), Elektromyogramm- (EMG) oder Elektroenzephalogrammdaten (EEG) messen und das Bestimmen des Zustands des Benutzers dies umfasst:
Verwenden der wenigstens zwei von EOG-, EKG-, EMG- oder EEG-Daten, um eine Überlastung im auditiven Cortex des Benutzers, eine Überlastung im okzipitalen Cortex des Benutzers, eine Blinzelrate des Benutzers oder Kieferbewegungen des Benutzers zu überwachen.

15. Verfahren zum Erhalten von biologisch relevanten Informationen, die mit einem Benutzer verknüpft sind, von einem am Körper tragbaren Audioprodukt (100), das einen elektroakustischen Wandler (108) aufweist, und zur Selbststeuerung des am Körper tragbaren Audioprodukts basierend auf den erhaltenen biologisch relevanten Informationen, umfassend:
Empfangen (202) elektrischer Signale von einer ersten Elektrode (102A, 102B, 102C), die über dem auditiven Cortex des Benutzers auf einer ersten Seite des Benutzerkörpers platziert ist;
Empfangen (204) elektrischer Signale von einer zweiten Elektrode (104A, 104B, 104C), die auf der ersten Seite des Benutzerkörpers platziert ist;
Empfangen (206) elektrischer Signale von einer ersten Referenzelektrode (106A, 106B, 106C), die auf der ersten Seite des Benutzerkörpers platziert ist;
Empfangen elektrischer Signale von einer dritten Elektrode, die über dem auditiven Cortex des Benutzers auf einer zweiten Seite des Benutzerkörpers platziert ist;
Empfangen elektrischer Signale von einer vierten Elektrode, die auf der zweiten Seite des Benutzerkörpers platziert ist;
Empfangen elektrischer Signale von einer zweiten Referenzelektrode, die auf der zweiten Seite des Benutzerkörpers platziert ist;
Verarbeiten eines kombinierten Signals von erstseitigen Bewegungsartefakten, dem Gehirn des Benutzers und der ersten Referenzelektrode;
Verarbeiten eines kombinierten Signals von zweitseitigen Bewegungsartefakten, dem Gehirn des Benutzers und Signalen von der zweiten Referenzelektrode;
Vergleichen des kombinierten Signals von der ersten Seite und dem kombinierten Signal von der zweiten Seite und Ausgeben einer Differenz pro Seite;
Reduzieren von Rauschen in den elektrischen Signalen, die von der ersten, zweiten, dritten und vierten Elektrode empfangen werden, wenigstens unter Verwendung der Differenz pro Seite;
Bestimmen eines Zustands des Benutzers unter Verwendung der rauschreduzierten Signale von der ersten, zweiten, dritten und vierten Elektrode; und
Durchführen (208) einer oder mehrerer Aktionen zum Steuern des Betriebs des am Körper tragbaren Audioprodukts basierend auf dem Zustand des Benutzers.

## Revendications

1. Produit audio portable (100) pour obtenir des informations biologiquement pertinentes associées à un utilisateur et pour se commander sur la base des informations biologiquement pertinentes obtenues, comprenant :
une première électrode (102A, 102B, 102C) placée sur le cortex auditif de l'utilisateur sur un premier côté du corps de l'utilisateur ;
une deuxième électrode (104A, 104B, 104C) placée sur le premier côté du corps de l'utilisateur ;
une première électrode de référence (106A, 106B, 106C) placée sur le premier côté du corps de l'utilisateur ;
une troisième électrode placée sur le cortex auditif de l'utilisateur sur un second côté du corps de l'utilisateur, la troisième électrode étant configurée pour surveiller des signaux ;
une quatrième électrode placée sur le second côté du corps de l'utilisateur ;
une seconde électrode de référence placée sur le second côté du corps de l'utilisateur ;
un processeur (102) couplé aux première, deuxième, troisième et quatrième électrodes, à la première électrode de référence et à la seconde électrode de référence, le processeur étant configuré pour :
traiter un signal combiné provenant des artefacts de mouvement du premier côté, du cerveau de l'utilisateur et de la première électrode de référence ;
traiter un signal combiné provenant des artefacts de mouvement du second côté, du cerveau de l'utilisateur et des signaux provenant de la seconde électrode de référence ;
comparer le signal combiné provenant du premier côté et le signal combiné provenant du second côté et délivrer une différence par côté ;
réduire le bruit dans les signaux reçus des première, deuxième, troisième et quatrième électrodes en utilisant au moins la différence par côté ;
déterminer un état de l'utilisateur à l'aide des signaux à bruit réduit provenant des première, deuxième, troisième et quatrième électrodes ; et
effectuer une ou plusieurs actions pour commander le fonctionnement du produit audio portable sur la base de l'état de l'utilisateur ; et
un transducteur électroacoustique (108) couplé au processeur.

2. Produit audio (100) selon la revendication 1, le fait d'effectuer l'action ou les actions pour commander le fonctionnement du produit audio portable comprenant l'une quelconque parmi : le changement d'un type de musique jouée, le changement d'un volume de musique jouée, l'accusé de réception d'une notification, le déclenchement d'une reconnaissance automatique de la parole.

3. Produit audio (100) selon la revendication 1, la deuxième électrode étant placée sur l'un parmi : le lobe occipital de l'utilisateur, l'arête du nez de l'utilisateur, ou dans un conduit auditif de l'utilisateur.

4. Produit audio (100) selon la revendication 1, la première électrode de référence étant placée sur le crâne de l'utilisateur derrière l'oreille de l'utilisateur.

5. Produit audio (100) selon la revendication 1, le processeur étant configuré pour réduire le bruit dans les signaux reçus des première, deuxième, troisième et quatrième électrodes par :
l'application d'une première boucle de rétroaction fermée pour des signaux reçus du premier côté du corps, la première rétroaction fermée se trouvant entre la première électrode de référence, d'une part, et la première électrode et la deuxième électrode, d'autre part ;
l'application d'une seconde boucle de rétroaction fermée pour des signaux reçus du second côté du corps, la seconde rétroaction fermée se trouvant entre la seconde électrode de référence, d'une part, et la troisième électrode et la quatrième électrode, d'autre part ; et
l'élimination du bruit des signaux reçus sur la base des première et seconde boucles de rétroaction fermées, l'élimination du bruit comprenant la corrélation croisée des signaux reçus du premier côté du corps avec les signaux reçus du second côté du corps.

6. Produit audio (100) selon la revendication 1, la détermination de l'état de l'utilisateur comprenant :
la détermination d'une chute d'impédance de signaux reçus d'au moins l'une parmi la première électrode, la deuxième électrode, la troisième électrode ou la quatrième électrode ; et
en réponse à la chute d'impédance déterminée, la détermination que l'utilisateur est stressé.

7. Produit audio (100) selon la revendication 6, le fait d'effectuer une ou plusieurs actions sur la base de l'état déterminé comprenant :
le suivi d'un type de musique jouée en réponse à la détermination que l'utilisateur est stressé ; et
en réponse à la détermination que l'utilisateur est stressé, la recommandation d'au moins l'un parmi une chanson ou une mélodie sur la base du type de musique suivi.

8. Produit audio (100) selon la revendication 1, au moins l'une des première, deuxième, troisième ou quatrième électrodes étant configurée pour mesurer des signaux d'électromyographie ou d'électrooculographie, et la détermination de l'état de l'utilisateur comprenant :
la détermination d'un taux de clignement diminué de l'utilisateur sur la base des signaux d'électromyographie ou d'électrooculographie reçus de l'au moins une des première, deuxième, troisième ou quatrième électrodes ; et
en réponse au taux de clignement diminué déterminé, la détermination que l'utilisateur est concentré.

9. Produit audio (100) selon la revendication 8, le fait d'effectuer une ou plusieurs actions sur la base de l'état déterminé comprenant :
en réponse à la détermination que l'utilisateur est concentré, la recommandation d'une certaine chanson ou mélodie.

10. Produit audio (100) selon la revendication 1, au moins l'une des première, deuxième, troisième ou quatrième électrodes étant configurée pour mesurer des signaux d'électromyographie, et la détermination de l'état de l'utilisateur comprenant :
la détermination de mouvements de la mâchoire sur la base des signaux d'électromyographie reçus de l'au moins une des première, deuxième, troisième ou quatrième électrodes ; et
à l'aide des signaux reçus du cortex auditif et les mouvements de la mâchoire déterminés, la détermination que l'utilisateur est en train de parler.

11. Produit audio (100) selon la revendication 10, le fait d'effectuer une ou plusieurs actions sur la base de l'état déterminé comprenant :
le réglage d'un volume de musique jouée par le produit audio en réponse à la détermination que l'utilisateur est en train de parler.

12. Produit audio (100) selon la revendication 10, comprenant en outre un microphone, le fait d'effectuer une ou plusieurs actions sur la base de l'état déterminé comprenant :
le déclenchement d'une reconnaissance automatique de la parole (ASR) par écoute à l'aide du microphone en réponse à la détermination que l'utilisateur est en train de parler.

13. Produit audio (100) selon la revendication 12, le fait d'effectuer une ou plusieurs actions sur la base de l'état déterminé comprenant en outre :
le passage dans un mode de faible consommation en réponse à la détermination que l'utilisateur n'est pas en train de parler.

14. Produit audio (100) selon la revendication 1, les première, deuxième, troisième et quatrième électrodes et les première et seconde électrodes de référence mesurant chacune au moins deux données parmi des données d'électrooculogramme (EOG),
d'électrocardiogramme (EKG), d'électromyogramme (EMG) ou d'électroencéphalogramme (EEG), et la détermination de l'état de l'utilisateur comprenant :
l'utilisation des au moins deux données parmi les données EOG, EKG, EMG ou EEG pour surveiller une surcharge dans le cortex auditif de l'utilisateur, une surcharge dans le cortex occipital de l'utilisateur, un taux de clignement de l'utilisateur ou des mouvements de la mâchoire de l'utilisateur.

15. Procédé pour obtenir des informations biologiquement pertinentes associées à un utilisateur à partir d'un produit audio portable (100) comprenant un transducteur électroacoustique (108), et pour que le produit audio portable se commande sur la base des informations biologiquement pertinentes obtenues, comprenant :
la réception (202) de signaux électriques provenant d'une première électrode (102A, 102B, 102C) placée sur le cortex auditif de l'utilisateur sur un premier côté du corps de l'utilisateur ;
la réception (204) de signaux électriques provenant d'une deuxième électrode (104A, 104B, 104C) placée sur le premier côté du corps de l'utilisateur ;
la réception (206) de signaux électriques provenant d'une première électrode de référence (106A, 106B, 106C) placée sur le premier côté du corps de l'utilisateur ;
la réception de signaux électriques provenant d'une troisième électrode placée sur le cortex auditif de l'utilisateur sur un second côté du corps de l'utilisateur ;
la réception de signaux électriques provenant d'une quatrième électrode placée sur le second côté du corps de l'utilisateur ;
la réception de signaux électriques provenant d'une seconde électrode de référence placée sur le second côté du corps de l'utilisateur ;
le traitement d'un signal combiné provenant des artefacts de mouvement du premier côté, du cerveau de l'utilisateur et de la première électrode de référence ;
le traitement d'un signal combiné provenant des artefacts de mouvement du second côté, du cerveau de l'utilisateur et des signaux provenant de la seconde électrode de référence ;
la comparaison du signal combiné provenant du premier côté et du signal combiné provenant du second côté et la délivrance d'une différence par côté ;
la réduction du bruit dans les signaux électriques reçus des première, deuxième, troisième et quatrième électrodes en utilisant au moins la différence par côté ;
la détermination d'un état de l'utilisateur à l'aide des signaux à bruit réduit provenant des première, deuxième, troisième et quatrième électrodes ; et
la prise (208) d'une ou plusieurs actions pour commander le fonctionnement du produit audio portable sur la base de l'état de l'utilisateur.
